# EUROPEAN PATENT SPECIFICATION

(11) **EP 1 791 589 B1**
(45) Date of publication and mention of the grant of the patent: **20.07.2011**
(21) Application number: 05764173.0
(22) Date of filing: 28.06.2005
(51) Int. Cl.: A61M 25/09

(54) **INTRAVASCULAR GUIDE WIRE**
INTRAVASCULÄRER FÜHRUNGSDRAHT
FIL DE GUIDAGE INTRAVASCULAIRE

(30) Priority: 31.08.2004 US 930458
(43) Date of publication of application: 06.06.2007
(73) Proprietor: Abbott Cardiovascular Systems Inc., Santa Clara, CA 95054 (US)
(72) Inventor: GRANDFIELD, Ryan c/o Advanced Cardiovascular Systems, Inc, Santa Clara, CA 95054-2807 (US); CORNISH, Wayne, E., Fallbrook, CA 92028 (US)
(74) Representative: McLeish, Nicholas Alistair Maxwell
(86) International application number: PCT/US2005/022708
(87) International publication number: WO 2006/025931

(56) References cited:
- EP-A- 1 388 350
- US-A1- 2004 167 443

## Description

### FIELD OF THE INVENTION

This invention relates to the field of medical devices, and more particularly to a guide wire for advancing a catheter within a body lumen in a procedure such as percutaneous transluminal coronary angioplasty (PTCA).

### BACKGROUND OF THE INVENTION

In a typical PTCA procedure, a guiding catheter having a preformed distal tip is percutaneously introduced into a patient's peripheral artery, e.g, femoral or brachial artery, by means of a conventional Seldinger technique and advanced therein until the distal tip of the guiding catheter is seated in the ostium of a desired coronary artery. A guide wire is first advanced by itself through the guiding catheter until the distal tip of the guide wire extends beyond the arterial location where the procedure is to be performed. Then a catheter is mounted onto the proximal portion of the guide wire which extends out of the proximal end of the guiding catheter which is outside of the patient. The catheter is advanced over the guide wire, while the position of the guide wire is fixed, until the operative element on the catheter is disposed within the arterial location where the procedure is to be performed. After the procedure is performed, the catheter may be withdrawn from the patient over the guide wire or the guide wire may be repositioned within the coronary anatomy for an additional procedure.

Conventional guide wires for angioplasty, stent delivery, atherectomy and other intravascular procedures usually have an elongate core member with one or more segments near the distal end thereof which taper distally to smaller cross sections. A flexible body member, such as a helical coil or a tubular body of polymeric material, is typically disposed about and secured to at least part of the distal portion of the core member. A shaping member, which may be the distal extremity of the core member or a separate shaping ribbon which is secured to the distal extremity of the core member, extends through the flexible body and is secured to the distal end of the flexible body by soldering, brazing or welding; or an adhesive may be used in the case of a polymeric flexible body which forms a rounded distal tip. The leading tip is highly flexible in order not to damage or perforate the vessel. The portion behind the distal tip becomes increasingly stiff, the better to support a balloon catheter or similar device.

For example, there is described in EP-A-1,388,350 a guide wire which includes a wire body and a coil provided so as to cover the distal side of the wire body. The wire body has a first wire disposed on the distal side, a second wire disposed on the proximal side from the first wire, and a third wire disposed on the proximal side from the second wire. The first wire is made from a reshapable metal material such as a stainless steel The second wire is made from a pseudoelastic alloy such as a Ni-Ti alloy. The third wire is made from a material having an elastic modulus larger than that of a material for forming the second wire. The first wire and the second wire are joined to each other by welding, and similarly the second wire and the third wire are joined to each other by welding.

A major requirement for guide wires is that they have sufficient column strength, to be pushed through a patient's vascular system or other body lumen without buckling. However, they must also be flexible enough to avoid damaging the blood vessel or other body lumen through which they are advanced. Efforts have been made to improve both the strength and flexibility of guide wires to make them more suitable for their intended uses, but these two properties are for the most part diametrically opposed to one another in that an increase in one usually involves a decrease in the other.

In order to fulfill these requirements, guide wires now typically include two different types or material joined together with a connecting tube, or sleeve, so that a proximal core will consist of a material having sufficient column strength and a distal core will be made of a flexible material to lead the advance through a body lumen. Currently, a nitinol hypotube or connecting tube is used as a sleeve to join a proximal stainless steel core to a nitinol distal core on certain types of guide wires. An example of this type of guide wire can be seen in, for example, U.S. Patent Nos. 6,248,082 and 6,602,208 (Jafari). The reason that an external tube is used to achieve the connection is because direct welding of nitinol to stainless steel has prove to be difficult if not effectively impossible. Attempts to achieve such a weld are met with serious deficiencies in the resulting strength and unique behavioral properties of nitinol. Furthermore, cracking may occur at the interface between the two metal portions at the weld However, when this problem is overcome by connection with an external connecting tube, the presence of the tube disadvantageously adds to the profile of the guide wire, tending to obstruct elements of the catheter that must slide along the guide wire during operation.

One prior solution to the general problem of connecting stainless steel to nitinol has been to insert an intermediate vanadium alloy transition piece between the stainless steel piece and the nitinol piece, welding the outer two metal pieces to the irmer transition piece. However, in the context of microwelding very small metal pieces, such as portions of a guide wire that may measure between about 1.02 and 0.25 mm (about 0.040 and 0.010 inches) diameter at the section to be joined, even this solution may cause deficiencies in the strength and behavioral properties of nitinol due to the high temperature required to melt vanadium It will be appreciated that welding small work pieces together provides less opportunity for heat to escape from the site of the weld, thus permitting heat buildup at the location of the weld to the detriment of the metal properties and the eventual uniformity and quality of the weld.

Thus, a need exists for an improved guide wire, and method for manufacture, that will address the needs of the prior art. It is believed that the present invention addresses these and other needs.

### SUMMARY OF THE INVENTION

The present invention provides an intravascular guide wire having the features set out in claim 1.

The present invention is directed to an intravascular guide wire having a stainless steel proximal portion joined to a nitinol distal portion without the use of an external tube or sleeve to reinforce the joint As noted above, it is known that direct welding of stainless steel to nitinol is difficult if not impossible, in that attempts to do so are met with serious deficiencies in the resulting weld strength and unique behavioral properties of nitinol.

Accordingly, in each embodiment of the present invention, a transition piece formed essentially of nickel is utilized to effect the connection between the stainless steel proximal portion and the nitinol distal portion, as it appears that nickel will form a welded bond with both stainless steel and nitinol, without cracking or metal property alteration taking place at the boundaries between the welded metals. The preferred composition of the transition piece is effectively pure nickel, although alloying with different mentals may be permitted to the extent that (a) this does not interfere with the ability of the resulting composition to form an essentially crack-free bond with the adjacent stainless steel and nitinol portions or (b) does not cause the melting temperature of me resulting composition to be elevated to a point where the heat required to form the weld removes or diminishes the unique characteristics of nitinol, In the preferred embodiment, welding may be performed by known methods of laser or friction welding, although other known forms of microwelding such as electron beam welding, and plasma arc welding may be used.

In different embodiments, the geometry of the transition piece may differ to provide different structural and strength characteristics and advantages, as desired. In a first embodiment, the transition piece has a simple cylindrical shape with flat ends that are normal to the guide wire longitudinal axis. In another embodiment, the ends of the cylindrical piece may be shaped to be convex or concave, to mate with the corresponding end faces of the proximal portion and the distal portion. Alternatively, flat ends of the cylindrical piece may be angled to the guide wire axis. In a further embodiment the transition piece may be shaped to be positioned between opposing end faces of the proximal and distal portions that are substantially parallel to the guide wire axis. In yet a further embodiment, the transition piece may be shaped to connect non-opposing end faces of the proximal and distal portions that are substantially parallel to the guide wire axis. Each of these alternative embodiments provides the opportunity to develop enhanced compressive, tension, and torsion strengths of the welded connection, by extending or reducing the length of the welded portion as needed. The overall torqueability and pushabiilty of the guide wire are thus improved over a conventional guide wire.

Further, the resulting connection has the advantage of not being positioned within a reinforcing sleeve, thereby reducing the outer profile of the guide wire at the position of the connection to permit unobstructed sliding of elements of the catheter surrounding the guide wire during operation.

These and other advantages of the invention will become more apparent from the following detailed description thereof and the accompanying exemplary drawings.

### BRIEF DESCRIPTION OF THE DRAWINGS

Figure 1 is a side elevational view of one embodiment of an intraluminal guide wire, showing features of the invention.
Fig. 2A is a fragmented perspective view of a portion of the guide wire of Fig. 1, showing a connection between proximal and distal portions via a cylindrical transition piece with flat ends.
Fig. 2B is a fragmented perspective view of a portion of the guide wire of Fig. 1, showing a connection between proximal and distal portions via a cylindrical transition piece with concave ends.
Fig. 2C is a fragmented perspective view of a portion of the guide wire of Fig. 1, showing a connection between proximal and distal portions via a cylindrical transition piece with convex ends.
Fig. 3 is a side elevational view of another embodiment of an intraluminal guide wire, showing features of the invention
Fig. 4 is a fragmented perspective view of a portion of the guide wire of Fig. 3, showing a connection between proximal and distal portions.
Fig. 5 is a side elevational view of a further embodiment of an intraluminal guide wire, showing features of the invention
Fig. 6 is a fragmented perspective view of a portion of the guide wire of Fig. 5, showing a connection between proximal and distal portions.
Fig. 7 is a side elevational view of yet a further embodiment of an intraluminal guide wire, showing features of the invention
Fig. 8 is a fragmented perspective view of a portion of the guide wire of Fig. 7, showing a connection between proximal and distal portions.

### DETAILED DESCRIPTION OF THE PREFERRED EMBODIMENTS

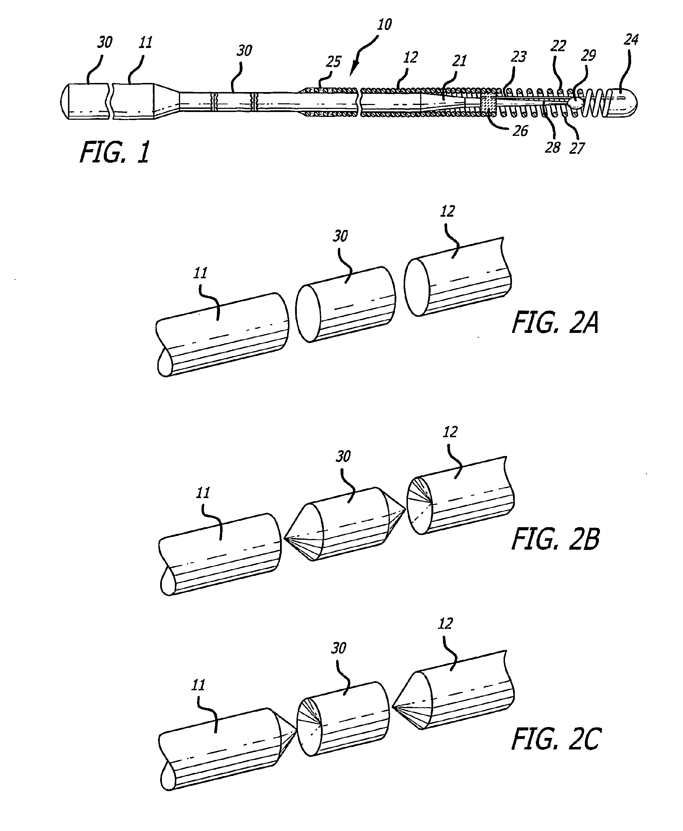

FIG. 1 lustrates a guide wire 10 embodying features, of the invention that is adapted to be inverted into a patient's body lumen, such as an artery or vein. The guide wire 10 comprises an elongated, relatively high strength proximal core section 11, and a relatively short flexible distal core section 12. The distal core portion 12 has at least one tapered section 21 which becomes smaller in the distal direction. A helical coil 22 is disposed about the distal core section 12 and is secured by its distal end to the distal end of shaping ribbon 23 by a mass of solder which forms rounded plug 24 when it solidifies. The proximal end of the helical coil 22 is secured to the distal core section 12 at a proximal location 25 and at intermediate location 26 by a suitable solder. The proximal end of the shaping ribbon 23 is secured to the distal core portion 12 at the same intermediate location 26 by the solder. Preferably, the most distal section 27 of the helical coil 22 is made of radiopaque metal, such as platinum or platinum-nickel alloy, to facilitate the fluoroscopic observation thereof while it is disposed within a patient's body. The most distal section 27 of the coil 22 should be stretched about 10 to about 30% in length to provide increased flexibility.

The most distal part 28 of the distal core section 12 is flattened into a rectangular cross-section and is preferably provided with a rounded tip 29, e.g., solder, to prevent the passage of the most distal part through the spacing between the stretched distal section 27 of the helical coil 22.

The exposed portion of the elongated proximal core section 11 should be provided with a coasting 30 of lubricous materials such as polytetrafluoroethylene (sold under the trademark TeflonⓇ by Du Pont, de Nemours & Co.) or other suitable lubricous coatings such as other fluoropolymers, hydrophilic coatings and polysiloxane coatings.

The elongated proximal core section 11 of the guide wire 10 is generally about 130 to about 140 cm in length with an outer diameter of about 0.15-0.45 mm (0.006 to 0.018 inch) for coronary use. Larger diameter guide wires, e.g. up to 0.89 mm (0.035 inch) or more may be employed m peripheral arteries and other body lumens. The lengths of the smaller diameter and tapered sections can range from about 1 to about 20 cm, depending upon the stiffness or flexibility desired in the final product The helical coil 22 may be about 3 to about 45 cm in length, preferably about 5 to about 20 cm, has an outer diameter about the same size as the outer diameter of the elongated proximal core section 11, and is made from wire about 0.025 to about 0.08 mm (0.001 - 0.003 Inch) in diameter typically about 0.05 mm (0.002 inch). The shaping ribbon 23 and the flattened distal section 28 of distal core section 12 have generally rectangularly shaped transverse cross-sections which usually have dimensions of about 0.013 to about 0.152 mm (0.0005 - 0.006 inch) preferably about 0.025 by 0.076 mm (0.001 - 0.003 inch).

The distal core section 12 is preferably made of nitinol, which is a psuedoelastic alloy material preferably consisting essentially of about 30 to about 52% titanium and the balance nickel and optionally up to 10% of one or more other alloying elements. The other alloying elements may be selected from the group consisting of iron, cobalt, vanadium, platinum, palladium and copper. The alloy can contain up to about 10% copper and vanadium and up to 3 % of the other alloying elements. The addition of nickel above the equiatomic amounts with titanium and the other identified alloying elements increases the stress levels at which the stress induced austenite-to-martensite transformation occurs and ensures that the temperature at which the martensitic phase thermally transforms to the austenitic phase is well below human body temperature (37 degrees C) so that austenite is the only temperature stable phase at body temperature. The excess nickel and additional alloying elements also help to provide an expanded strain range at very high stresses when the stress induced transformation of the austenitic phase to the martensitic phase occurs. Moreover, it is known that heating nitinol excessively can change the pseudoelastic behavior, the martensite transitions temperatures, and even the shape memory. Therefore, heat input into the nitinol should be carefully controlled.

One method for making the pseudoelastic distal core section is to cold work, preferably by drawing, a rod having a composition according to the relative proportions described above and then heat treating the cold worked product while it is under stress to impart a shape memory thereto. Typical initial transverse dimensions of the rod are about 1.14 mm to about 6.35 mm (about 0.045 inch to about 0.25 inch). Before drawing the solid rod, it is preferably annealed at a temperature of about 500 to about 750 degrees C, typically about 650 degrees C, for about' 30 minutes in a protective atmosphere such as argon to relieve essentially all internal stresses. In this manner all of the specimens start the subsequent thermomechanical processing in essentially the same metallurgical condition so that products with consistent final properties are obtained. Such treatment also provides the requisite ductility for effective cold working.

The stress-relieved stock is cold worked by drawing in order to effect a reduction in the cross sectional area thereof of about 30 to about 70%. The metal is drawn through one or more dies of appropriate inner diameter with a reduction per pass of about 10% to 50%. Other forms of cold working can be employed such as swaging.

Following cold work, the drawn wire product is heat treated at a temperature between about 350 degrees C and about 600 degrees C for about 0.5 to about 60 minutes. Preferably, the drawn wire product is simultaneously subjected to a longitudinal stress between about 5% and about 50%, preferably about 10% to about 30% of the tensile strength of the material (as measured at room temperature) in order to impart a straight "memory" to the metal and to ensure that any residual stresses therein are uniform. This memory imparting heat treatment also fixes the austenite-martensite transformation temperature for the cold worked metal. By developing a straight "memory" and maintaining uniform residual stresses in the pseudoelastic material, there is little or no tendency for a guide wire made of this material to whip when it is torqued within a patient's blood vessel. The term "whip" refers to the sudden rotation of the distal tip of a guide wire when the proximal end of the guide wire is subjected to torque.

An alternative method for imparting a straight memory to the cold worked material includes mechanically straightening the wire and then subjecting the straightened wire to a memory imparting heat treatment at a temperature of about 300 degrees to about 450 degrees C, preferably about 330 degrees C to about 400 degrees C The latter treatment provides substantially improved tensile properties, but it is not very effective on materials which have been cold worked above 55%, particularly above 60%. Materials produced in this manner exhibit stress-induced austenite to martensite phase transformation at very high levels of stress but the stress during the phase transformation is not nearly as constant as the previously discussed method. Conventional mechanical straightening means can be used such as subjecting the material to sufficient longitudinal stress to straighten it.

Because of the extended strain range under stress-induced phase transformation which is characteristic of the pseudoelastic material described herein, a guide wire having a distal portion made at least in substantial part of such material can be readily advanced through tortuous arterial passageways. When the distal end of the guide wire engages the wall of a body lumen such as a blood vessel, it will pseudoelastically deform as the austenite transforms to martensite. Upon the disengagement of the distal end of the guide wire from the vessel wall, the stress is reduced or eliminated from within the pseudoelastic portion of the guide wire and it recovers to its original shape, i.e., the shape "remembered" which is preferably straight. The straight "memory" in conjunction with little or no nonuniform residual longitudinal stresses within the guide wire prevent whipping of the guide wire's distal end when the guide wire is torqued from the proximal end thereof. Moreover, due to the very high level of stress needed to transform the austenite phase to the martensite phase, there is little chance for permanent deformation of the guide wire or the guiding member when it is advanced through a patient's artery.

The present invention provides a guide wire which exhibits, at the distal portion, pseudoelastic characteristics to facilitate the advancement thereof in a body lumen. The distal guiding portion exhibits extensive, recoverable strain resulting from reversible, stress induced phase transformation of austenite to martensite at exceptionally high stress levels which greatly minimizes the risk of damage to arteries during the advancement therein.

The high strength proximal portion of the guide wire generally is significantly stronger, i.e., higher ultimate tensile strength, than the pseudoelastic distal portion. Suitable high strength materials include 304 stainless steel which is a conventional material in guide wire construction. Other high strength materials include nickel- cobalt- molybdenum-chromium alloys such as commercially available MP35N alloy.

Turning now to the connection between the stainless steel proximal portion 11 and the nitinol distal portion 12 of the guide wire, it has been found that connecting these two portions together by welding each to opposite ends of an intermediate transition piece formed from nickel achieves the desired connection without causing deficiencies in the strength and behavioral properties of the distal nitinol portion. While effectively unalloyed nickel is preferred for the transition piece, alloying the nickel with, for example, titanium, cobalt, copper or iron, to a degree which does not alter its ability to continuously form an essentially crack-free welded bond with the stainless steel proximal portion and nitinol distal portion, is permissible under alternative embodiments.

In a preferred embodiment, exemplified in FIGS. 1 and 2, a butt weld may be used at each end of the transition piece 30 which may be cylindrically shaped. The transition piece 30 advantageously may have an aspect ratio (i.e., ratio of length to diameter) of between 0.5 and 3, preferably greater than 1.0. Furthermore, as seen in FIGS. 2B and 2C, the transition piece 30 may have a conical or a dome shaped end that is convex or concave. Likewise, the interface surface of the proximal or distal portion 11,12 has a complementary mating shape. Welding may be achieved by known methods of microwelding, such as friction welding, laser welding, electron beam welding, and plasma arc welding. Examples of known welding methods are described in U.S. Patent 6,729,526 (friction welding), U.S. Patent 4,358,658 (laser welding), and U.S. Patent 5,951,886 (electron beam welding). In one preferred embodiment, friction welding is preferred as providing a high degree of precision and control. In another preferred embodiment, laser welding may be preferred as also providing a high degree of precision and control.

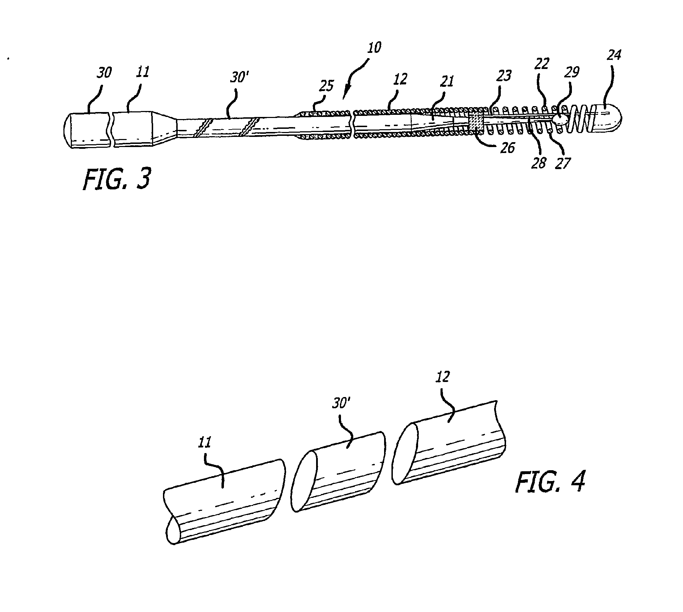

In an alternative embodiment, exemplified in FIGS. 3 and 4, transition piece 30' may be shaped to contact the outer metal portions 11, 12 at an angle oblique to the longitudinal guide wire axis between about 30 degrees and 60 degrees, preferably 45 degrees, to provide a larger area of contact for opposing welded surfaces. It will be appreciated that friction welding may not be possible under these conditions, but laser welding will be a preferred method, giving rise to a connection with greater surface contact between the welded parts than the previous embodiment, and thus greater tensile, compressive, and torsional resistance characteristics.

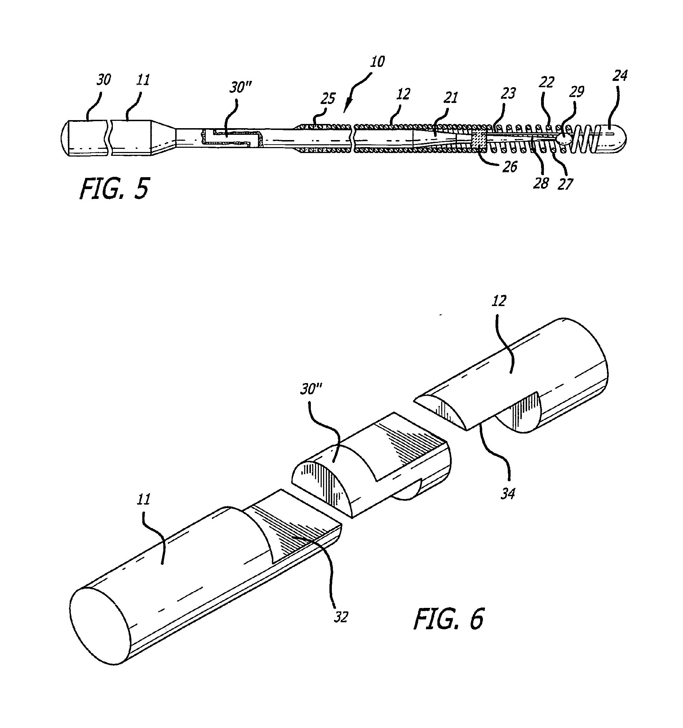

In a further alternative embodiment, exemplified in FIGS. 5 and 6, the transition piece 30" may be shaped to fit between the outer metal portions 11, 32 which are shaped to provide a connection substantially between a horizontal surface 32 of the proximal portion and an opposing horizontal surface 34 of the distal portion. This configuration may be adapted to have the advantage of providing an even larger area of contact between the juxtaposed parts than that of the embodiment of FIGS. 3 and 4. A profile view of the transition piece 30" gives the appearance of a zigzag shape.

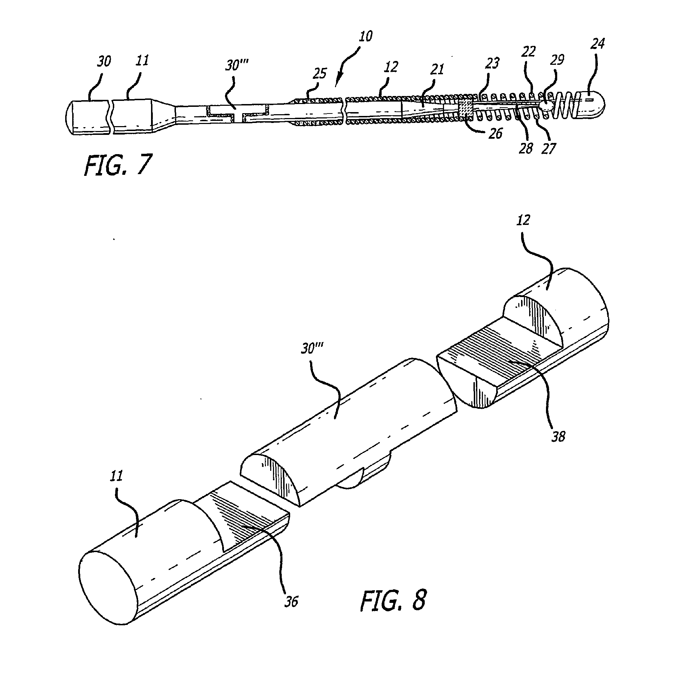

In yet a further alternative embodiment, exemplified in FIGS. 7 and 8 (with similar advantages of the embodiment of FIGS. 5 and 6), the transition piece 30"' may be shaped to connect the outer metal portions 11, 12 which have in turn been shaped to provide a connection between a horizontal surface 36 of the proximal portion 11 and an adjacent non-opposing horizontal surface 38 of the distal portion 12. A profile view of the transition piece 30" gives the appearance of a "T" shape. It will be appreciated that a combination of the various features of transition piece 30, 30', 30" and 30'" may be used.

After the proximal and distal portions are thus connected, the guide wire may be cleaned in the vicinity of the connection by known means such as electropolishing brushing, or grinding to remove any slag or minor rough spots.

An advantageous characteristic arising from forming the transition piece 30 of nickel, or a mild alloy of nickel, is that, compared with vanadium which is known to be a successful transition piece for welding stainless steel to nitinol generally, nickel has a lower melting point than vanadium. Thus, the microwelding process would tend to impart less heat to the distal portion of the guide wire than vanadium would requite, and is therefore more suitable for microwelding as it is less likely to alter the beneficial characteristics of the nitinol alloy (such as the amount of pseudo-elasticity and the phase transition temperatures) in the process of welding.

Another advantageous feature of nickel is that it has a higher coefficient of thermal expansion than vanadium, and thus is better matched with the higher coefficient of thermal expansion of the stainless steel proximal portion, and of the distal nitinol portion. Accordingly, during healing or cooling of the weld in this case, less volumetric expansion or contraction differential may occur at the boundaries between the transition piece and the proximal and distal portions, and consequently, there is less tendency for cracking or locked-in stresses to form at the boundaries.

The resulting guide wire presents a uniform outer profile, allowing free movement of catheter elements along the guide wire during operation. In the context of microwelding workpieces as small as those of an intraluminal guide wire (i.e., less than 9.02 mm [0.040 inches]) the solution of interposing a welded transition piece formed essentially of nickel between a stainless steel portion and a nitinol portion achieves adequate strength and flexibility.

While a particular form of the invention has been illustrated and described, it will also be apparent to those skilled in the art that various modifications can be mad without departing from the scope of the invention. Accordingly, it is not intended that the invention be limited except by the appended claims.

## Claims

1. An intravascular guide wire (10), comprising:
a proximal guide wire core portion (11) with proximal and distal ends made of stainless steel;
a distal portion (12) with proximal and distal ends made of a psuedoelastic metal alloy consisting essentially of about 30% to about 52% titanium and-the balance nickel and up to 10% of one or more other alloying elements; and
a transition piece (30) with proximal and distal ends made essentially of nickel;
wherein the distal end of the proximal portion (11) is welded to the proximal end of the transition piece (30), and the distal end of the transition piece (30) is welded to the proximal end of the distal portion (12).

2. The intravascular guide wire (10) of claim 1, wherein the transition piece (30) is cylindrically shaped having a length greater than a diameter thereof.

3. The intravascular guide wire (10) of claim 2, wherein the transition piece (30) has an aspect ratio of between 0.5 and 3.

4. The intravascular guide wire (10) of claim 2, wherein the ends of the cylindrically shaped transition piece (30) are angled obliquely to the axis of the guide wire (10).

5. The intravascular guide wire of claim 4, wherein the ends of the cylindrically shaped transition piece (30) are angled at between 30 and 60 degrees to the axis of the guide wire (10).

6. The intravascular guide wire (10) of claim 1. wherein the transition piece (30) has a cylindrical shape with at least one of a convex and a conical end.

7. The intravascular guide wire (10) of claim 1, wherein the transition piece (30") is configured to connect opposing faces (32,34) of the proximal and distal portions (11.12), the opposing faces (32,34) being substantially parallel to the axis of the guide wire (10).

8. The intravascular guide wire (10) of claim 1, wherein the transition piece (30"') is configured to connect non-opposing faces (36,38) of the proximal and distal portions (11,12), the non-opposing faces (36,38) being substantially parallel to the axis of the guide wire (10).

9. The intravascular guide wire (10) of claim 1, wherein the transition piece (30",30"') has at least one of a zigzag and a T shaped profile.

10. The intravascular guide wire (10) of claim 1, wherein the transition piece (30) is not covered by a sleeve.

11. The intravascular guide wire (10) of claim 1, wherein the welding is achieved by friction welding.

12. The intravascular guide wire (10) of claim 1, wherein the welding is achieved by laser welding.

13. The intravascular guide wire (10) of claim 1, wherein the transition piece (30) has a length greater than a diameter thereof; is micro-welded coaxially in between the distal and proximal sections (11,12); and does not include an outer sleeve.

## Patentansprüche

1. lntravaskulärer Führungsdraht (10), umfassend:
einen proximalen Führungsdraht-Kernabschnitt (11) mit einem proximalen und
einem distalen Ende aus Edelstahl;
einen distalen Abschnitt (12) mit einem proximalen und einem distalen Ende aus einer pseudoelastischen Metalllegierung, die im Wesentlichen ungefähr 30% bis ungefähr 52% Titan und als Rest Nickel sowie bis zu 10% eines oder mehrerer anderer Legierungselemente umfasst; und
ein Übergangsstück (30) mit einem proximalen und einem distalen Ende, das im Wesentlichen aus Nickel gebildet ist;
wobei das distale Ende des proximalen Abschnitts (11) an das proximale Ende des Übergangsstücks (30) geschweißt ist und das distale Ende des Übergangsstücks (30) an das proximale Ende des distalen Abschnitts (12) geschweißt ist.

2. Intravaskulärer Führungsdraht (10) nach Anspruch 1, wobei das Übergangsstück (30) zylindrisch geformt ist und eine Länge aufweist, die größer ist als sein Durchmesser.

3. Intravaskulärer Führungsdraht (10) nach Anspruch 2, wobei das Übergangsstück (30) ein Längenverhältnis zwischen 0,5 und 3 aufweist.

4. Intravaskulärer Führungsdraht (10) nach Anspruch 2, wobei die Enden des zylindrisch geformten Übergangsstücks (30) zu der Achse des Führungsdrahts (10) schräg abgewinkelt sind.

5. Intravaskulärer Führungsdraht (10) nach Anspruch 4, wobei die Enden des zylindrisch geformten Übergangsstücks (30) zu der Achse des Führungsdrahts (10) um zwischen 30 und 60 Grad abgewinkelt sind.

6. Intravaskulärer Führungsdraht (10) nach Anspruch 1, wobei das Übergangsstück (30) eine zylindrische Form mit wenigstens einem von konvexem oder konischem Ende aufweist.

7. Intravaskulärer Führungsdraht (10) nach Anspruch 1, wobei das Übergangsstück (30") dazu ausgebildet ist, gegenüberliegende Flächen (32,34) des proximalen und distalen Abschnitts (11,12) zu verbinden, wobei die gegenüberliegenden Flächen (32,34) im Wesentlichen parallel zu der Achse des Führungsdrahts (10) sind.

8. Intravaskulärer Führungsdraht (10) nach Anspruch 1, wobei das Übergangsstück (30"') dazu ausgebildet ist, nicht gegenüberliegende Flächen (36,38) des proximalen und distalen Abschnitts (11,12) zu verbinden, wobei die nicht gegenüberliegenden Flächen (36,38) im Wesentlichen parallel zu der Achse des Führungsdrahts (10) sind.

9. Intravaskulärer Führungsdraht (10) nach Anspruch 1, wobei das Übergangsstück (30", 30'") wenigstens eines von zickzackförmigem oder T-förmigem Profil aufweist.

10. lntravaskulärer Führungsdraht (10) nach Anspruch 1, wobei das Übergangsstück (30) nicht von einer Hülle bedeckt ist.

11. Intravaskulärer Führungsdraht (10) nach Anspruch 1, wobei das Schweißen durch Reibschweißen erzielt wird.

12. lntravaskulärer Führungsdraht (10) nach Anspruch 1, wobei das Schweißen durch Laserschweißen erzielt wird.

13. Intravaskulärer Führungsdraht (10) nach Anspruch 1, wobei das Übergangsstück eine Länge besitzt, die größer ist als sein Durchmesser, zwischen dem distalen und dem proximalen Abschnitt (11,12) koaxial mikroverschweißt ist und keine Außenhülle umfasst.

## Revendications

1. Fil guide intravasculaire (10), comprenant :
une partie (11) d'âme proximale du fil guide avec des extrémités proximale et distale réalisées en acier inoxydable ;
une partie distale (12) avec des extrémités proximale et distale réalisées en alliage métallique pseudo-élastique constitué essentiellement d'environ 30 % à environ 52 % de titane et le reste étant le nickel et jusqu'à 10 % d'un ou de plusieurs autres éléments d'alliage ; et
une pièce (30) de transition avec des extrémités proximale et distale réalisées essentiellement en nickel ;
où l'extrémité distale de la partie proximale (11) est soudée à l'extrémité proximale de la pièce de transition (30), et l'extrémité distale de la pièce de transition (30) est soudée à l'extrémité proximale de la partie distale (12).

2. Fil guide intravasculaire (10) de la revendication 1, dans lequel la pièce de transition (30) présente une forme cylindrique dont la longueur est supérieure à son diamètre.

3. Fil guide intravasculaire (10) de la revendication 2, dans lequel la pièce de transition (30) présente un rapport longueur/diamètre entre 0,5 et 3.

4. Fil guide intravasculaire (10) de la revendication 2, dans lequel les extrémités de la pièce (30) de transition de forme cylindrique sont inclinées en biais par rapport à l'axe du fil guide (10).

5. Fil guide intravasculaire de la revendication 4, dans lequel les extrémités de la pièce (30) de transition de forme cylindrique sont inclinées entre 30 et 60 degrés par rapport à l'axe du fil guide (10).

6. Fil guide intravasculaire (10) de la revendication 1, dans lequel la pièce de transition (30) a une forme cylindrique avec au moins l'une parmi une extrémité convexe et une extrémité conique.

7. Fil guide intravasculaire (10) de la revendication 1, dans lequel la pièce de transition (30") est configurée pour relier des faces opposées (32,34) des parties proximale et distale (11, 12), les faces opposées (32,34) étant sensiblement parallèles à l'axe du fil guide (10).

8. Fil guide intravasculaire (10) de la revendication 1, dans lequel la pièce de transition (30"') est configurée pour relier des faces non-opposées (36, 38) des parties proximale et distale (11, 12), les faces non-opposées (36, 38) étant sensiblement parallèles à l'axe du fil guide (10).

9. Fil guide intravasculaire (10) de la revendication 1, dans lequel la pièce de transition (30", 30"') a au moins l'un d'un profil en zigzag et en forme de T.

10. Fil guide intravasculaire (10) de la revendication 1, dans lequel la pièce de transition (30) n'est pas couverte par une gaine.

11. Fil guide intravasculaire (10) de la revendication 1, dans lequel la soudure est réalisée par soudage par friction.

12. Fil guide intravasculaire (10) de la revendication 1, dans lequel la soudure est réalisée par soudage laser.

13. Fil guide intravasculaire (10) de la revendication 1, dans lequel la pièce de transition (30) a une longueur supérieure à son diamètre ; est micro-soudée de manière coaxiale entre les tronçons distal et proximal (11, 12) ; et ne comporte pas une gaine extérieure.
